# EUROPEAN PATENT APPLICATION

(11) **EP 3 251 664 A1**
(43) Date of publication of application: **06.12.2017**
(21) Application number: 16172714.4
(22) Date of filing: 02.06.2016
(51) Int. Cl.: A61K 31/00

(54) **CYSTEINE DIOXYGENASE INHIBITORS FOR PROLONGING THE LIFESPAN OF A SUBJECT**

(71) Applicant: RIJKSUNIVERSITEIT GRONINGEN, 9712 CP Groningen (NL); ACADEMISCH ZIEKENHUIS GRONINGEN, 9713 GZ Groningen (NL)
(72) Inventor: Nollen, Ellen Alexandra Adriënne, 9713 AV Groningen (NL); Michels, Helen, 9713 AV Groningen (NL)
(74) Representative: V.O.

(57) **Abstract**

The invention relates compositions and methods of prolonging the lifespan of a subject or providing anti-aging benefits. A method of the invention comprises administering an agent that inhibits the conversion of cysteine to cysteine sulfinic acid to a subject, for example using an inhibitor of cysteine dioxygenase (CDO). A composition of the invention may comprise a) a CDO inhibitor; and b) a second agent known to be useful in prolonging the lifespan of a subject or preventing or reducing age-related symptoms. Also provided is a CDO-based screening method for identifying a compound capable of prolonging the lifespan of a subject or preventing or reducing age-related symptoms in a subject.

## Description

The present invention relates to novel therapeutic targets for aging. In particular, the present invention provides compositions and methods of prolonging the lifespan of a subject or providing anti-aging benefits.

Lifespan regulation is a complex process that is coordinated by divergent pathways, including DNA damage, oxidative stress, and metabolic networks (Houtkooper et al., 2010, Cell 142, 9). Gain- and loss-of-function studies in animal models including mice, *C. elegans* and D. melanogaster, have shown that caloric excess pathways, such as the insulin/IGF1 signaling pathway and the mammalian target of rapamycin (mTOR), decrease lifespan (Harrison et al., 2009, Nature 460, 392-395; Bjedov et al., 2010, Cell Metab 11, 35; Zid et al., 2009, Cell 139, 149; Selman et al., 2009, Science 326, 140), whereas the caloric restriction pathways, for instance AMP-activated protein kinase (AMPK) and sirtuins, tend to increase lifespan (Mair et al., 2011, Nature 470, 404-408; Anisimov et al., 2008, Cell Cycle 7, 2769). Whether or not such genes also contribute to natural lifespan is, however, not understood.

Aging and the diseases associated with it are a heavy burden on society. The current increase in life expectancy not only impacts on our social systems, but also goes hand in hand with the emergence of common chronic diseases, including those of the nervous, immune, and cardio-metabolic systems, which often reach epidemic proportions. In this respect it is important to understand the natural aging process and to elucidate where lifestyle and/or pharmacological interventions can have an impact. In recent years, many novel therapeutic options have been suggested to prevent aging-associated diseases. Although some of these pharmaceutical interventions were shown to extend lifespan, even in mammals (Harrison et al., 2009, supra; Pearson et al., 2008, Cell Metab 8, 157-168), there is still a need for identification of novel compounds that have the ability to increase lifespan and, thus, be useful for delaying aging and/or preventing or treating age-related diseases and disorders.

Accordingly, the present inventors aimed at finding novel "metabolic drivers" of aging. In particular, it was found that depletion of the enzyme cysteine dioxygenase (*cdo-1*), which catalyzes the conversion of cysteine to sulfinic acid, increased the lifespan of *C*. *elegans* and furthermore improved motility during aging. The catalytic conversion of cysteine to sulfinic acid is herewith shown to be a novel target for regulate longevity. Surprisingly, this effect was also seen when the gene was knocked down at day one of adulthood, after development is complete. Furthermore, knockdown of *cdo-1* did not cause any visible unwanted side effects in the animals.

Accordingly, in one embodiment, the invention provides a method of prolonging the lifespan of a subject or preventing or reducing one or more age-related symptoms, comprising administering an agent that inhibits the conversion of cysteine to cysteine sulfinic acid to a subject, wherein said administering prolongs the lifespan of said subject relative to the lifespan in the absence of said agent or wherein age-related symptoms are reduced or prevented.

For example, in one aspect the invention provides a method of increasing lifespan in a subject, comprising administering to the subject an agent that inhibits the conversion of cysteine to cysteine sulfinic acid.

In a second aspect, the invention relates to a method of inhibiting or delaying the aging process in a subject comprising administering to the subject an agent that inhibits the conversion of cysteine to cysteine sulfinic acid.

In a third aspect, the invention relates to a method of treating, preventing, or delaying, an age-related disease or disorder or a mitochondrial disease or disorder, in a subject, comprising administering to the subject an agent that inhibits the conversion of cysteine to cysteine sulfinic acid.

A fourth aspect of the invention resides in an agent or composition that that inhibits the conversion of cysteine to cysteine sulfinic acid, including for instance by inhibiting the mitochondrial translation machinery function in a subject, for use in increasing lifespan, inhibiting or delaying the aging process, and/or treating, preventing, or delaying, an age-related disease or disorder or a mitochondrial disease or disorder, in said subject.

A fifth aspect of the invention relates to a method of screening a compound for its ability to increase lifespan, inhibit or delay the aging process, and/or prevent, treat, or delay an age-related disease or disorder or a mitochondrial disease or disorder, in a subject, comprising:
a) contacting a cell or a non-human subject with a test compound; and
b) measuring the conversion of cysteine to cysteine sulfinic acid in the presence and in the absence of the test compound in said cell or said non-human subject,
wherein a test compound that inhibits the conversion of cysteine to cysteine sulfinic acid determined in step b) in the cell or non-human subject indicates a compound that is able to increase lifespan, inhibit or delay the aging process, and/or prevent, treat, or delay an age-related disease or disorder or a mitochondrial disease or disorder.

A sixth aspect relates to a compound for use in a method of prolonging the lifespan of a subject or preventing or reducing age-related symptoms in a subject, said method comprising administering to said subject an agent that inhibits the conversion of cysteine to cysteine sulfinic acid, wherein said administering prolongs the lifespan of said subject relative to the lifespan in the absence of said agent or wherein age-related symptoms are reduced or prevented.

US2013/101580 also relates to therapeutic targets for aging. In particular, it discloses the inhibition of the kynurenine pathway of tryptophan metabolism to extend lifespan or provide anti-aging benefits.

A role of inhibiting the pathway involving the conversion of cysteine to cysteine sulfinic acid, e.g. by inhibition of cysteine dioxygenase (CDO in humans), in ageing or lifespan regulation has not been disclosed or suggested in the art. In contrast, the prior art provides a teaching away from the present invention by implicating elevated cysteine levels and down-regulated CDO expression in the pathogenesis of age-related disorders like Parkinson's disease.

See in particular Questi et al., (Cell Biol Toxicol. 2000;16(4):243-55) disclosing an *in vitro* model for CDO expression in the brain. It is explicitly mentioned that a decrease in CDO activity may give rise to an increase in cysteine levels, resulting in cell death mediated either by cytotoxicity or by conjugation with dopamine.

Ye et al. (J. Biol. Chem. 2007, VOL. 282, NO. 5, pp. 3391-3402) teaches that homocysteine is a natural competitive inhibitor of CDO. They also refer to studies which link high levels of homocysteine to cardiovascular disease and neurodegenerative diseases. It is suggested that "homocysteine may be a lead compound to target CDO for future therapeutic applications", presumably by finding a drug that interferes with endogenous homocysteine to alleviate CDO inhibition. Other articles also implicate elevated cysteine to have detrimental effects in neurodegenerative diseases. See e.g. Heafield (Neuroscience Letters, 110 (1990) 21 6-220)).

In view of the above, it is clear that the prior art fails to provide an incentive to use an inhibitor of CDO, thus decreasing conversion of intracellular cysteine to cysteine sulfinic acid, in the treatment of age-related (neurodegenerative) diseases.

In some embodiments, the present invention comprises administering an agent that inhibits the conversion of cysteine to cysteine sulfinic acid to a subject, wherein the administering prolongs the lifespan of the subject relative to the lifespan in the absence of the agent or where the one or more anti-aging benefits are achieved.

Also provided herein is a compound for use in a method of prolonging the lifespan of a subject or preventing or reducing age-related symptoms in a subject, said method comprising administering to said subject an agent that inhibits the conversion of cysteine to cysteine sulfinic acid, wherein said administering prolongs the lifespan of said subject relative to the lifespan in the absence of said agent or wherein age-related symptoms are reduced or prevented.

A further aspect relates to a pharmaceutical composition comprising an agent that inhibits the conversion of cysteine to cysteine sulfinic acid, and a pharmaceutically acceptable carrier, diluent or vehicle.

In one embodiment, the agent inhibits cysteine dioxygenase (CDO). For example, in some embodiments, the agent is a small molecule, a nucleic acid (e.g., siRNA or antisense nucleic acid), an antibody, etc. In some embodiments, the administering treats, prevents reduces or retards one or more aging-associated medical or psychiatric disorders or conditions. For example, the disorder is a neurodegenerative disorder. In a specific aspect, the disorder is Huntington's disease, Parkinson's disease or Alzheimer's disease.

In some embodiments, the present invention provides a composition comprising an agent that inhibits the conversion of cysteine to cysteine sulfinic acid; and a secondary agent known to be useful in treating one or more disorders associated with aging. In some embodiments, the agents are formulated in a single pharmaceutical composition.

As used herein, the term "prolonging the lifespan of a subject" refers to increasing the lifespan of a subject relative to the lifespan in the absence of the agent (e.g., relative to the projected lifespan of an individual, a population, an individual with certain diseases or lifestyle choices, etc.).

As used herein, the term "inhibits the conversion of cysteine to cysteine sulfinic acid" refers to any method of inhibiting the conversion of cysteine to cysteine sulfinic acid. In some embodiments, the enzyme catalyzing cysteine conversion to cysteine sulfinic acid (e.g., CDO) is inhibited. CDO may be inhibited using any suitable agent (e.g., via directly contacting CDO, contacting CDO mRNA or genomic DNA, causing conformational changes of CDO polypeptide, decreasing CDO protein levels, or interfering with CDO interactions with signaling partners, and/or affecting the expression of CDO target genes). Inhibitors also include molecules that indirectly regulate CDO biological activity by intercepting upstream signaling molecules.

As used herein, the term "subject" refers to any animal (e.g., a mammal), including, but not limited to, humans, non-human primates, rodents, and the like, which is to be the recipient of a particular treatment. Typically, the terms "subject" and "patient" are used interchangeably herein in reference to a human subject.

As used herein, the term "non-human animals" refers to all non-human animals including, but are not limited to, vertebrates such as rodents, non-human primates, ovines, bovines, ruminants, lagomorphs, porcines, caprines, equines, canines, felines, ayes, etc.

Also provided herein is a method of identifying a compound capable of prolonging the lifespan of a subject or preventing or reducing age-related symptoms in a subject, comprising the step of a) contacting a cell with a test compound; and b) identifying a test compound that inhibits the conversion of cysteine to cysteine sulfinic acid. Preferably, this screening method comprises determining whether the test compound is capable of inhibiting cysteine dioxygenase (CDO). For example, identifying a test compound that inhibits the conversion of cysteine to cysteine sulfinic acid comprises identifying a compound that can inhibit at least 10%, preferably at least 20%, more preferably at least 30% of the activity of CDO.

In one embodiment, the invention provides a method of screening a compound for its ability to increase lifespan, inhibit or delay the aging process, and/or prevent, treat, or delay an age-related disease or disorder or a mitochondrial disease or disorder, in a subject, comprising:
a) contacting a cell or a non-human subject with a test compound; and
b) measuring the conversion of cysteine to cysteine sulfinic acid in the presence and in the absence of the test compound in said cell or said non-human subject,
wherein a test compound that inhibits the conversion of cysteine to cysteine sulfinic acid determined in step b) in the cell or non-human subject indicates a compound that is able to increase lifespan, inhibit or delay the aging process, and/or prevent, treat, or delay an age-related disease or disorder or a mitochondrial disease or disorder.

In one embodiment, the screening method is an *in vitro* method using a mammalian cell, preferably a human cell.

In another embodiment, the screening method is performed using a cell which is in an animal. In some embodiments, the cell is *C. elegans, Drosophila* or a non-human mammal. Preferably, the cell is in *C. elegans.*

The method preferably comprises measuring CDO-related metabolites (e.g. cysteine and/or cysteine sulfinic acid) that can be used to validate functional inhibition of CDO. In a specific embodiment, it comprises a fluorescence-based assay to detect CDO-related metabolites in cells that can be used for high throughput screening. A CDO knock out cell or cell line is suitably used to validate the effects of a compound, excluding an indirect effect on cellular functions independently from CDO.

As will be appreciated by the skilled person, a screening method of the invention can be used to identify any class or type of (therapeutically) relevant compound. For example, the test compound may be selected from the group consisting of small molecules, nucleic acids and antibodies. In some embodiments, the agent is a small molecule, a nucleic acid (e.g., siRNA or antisense nucleic acid), an antibody, etc. In some embodiments, two or more agents working together (e.g., additively or synergistically) are evaluated. In some embodiments, aging-associated medical disorders (e.g., aging-associated medical and psychiatric disorders) include, but are not limited to, reduced cardiac output, atherosclerosis, high blood pressure, mood & cognitive changes, memory loss, vision problems, hearing loss, muscle wasting, decreased energy, abdominal fat/truncal obesity, weak bones, problems with coordination and balance, digestive problems (e.g., constipation), urinary incontinence, diabetes, thin skin and skin wrinkles, poor sleep, age related mobility problems and lessened sexual performance.

### LEGEND TO THE FIGURE

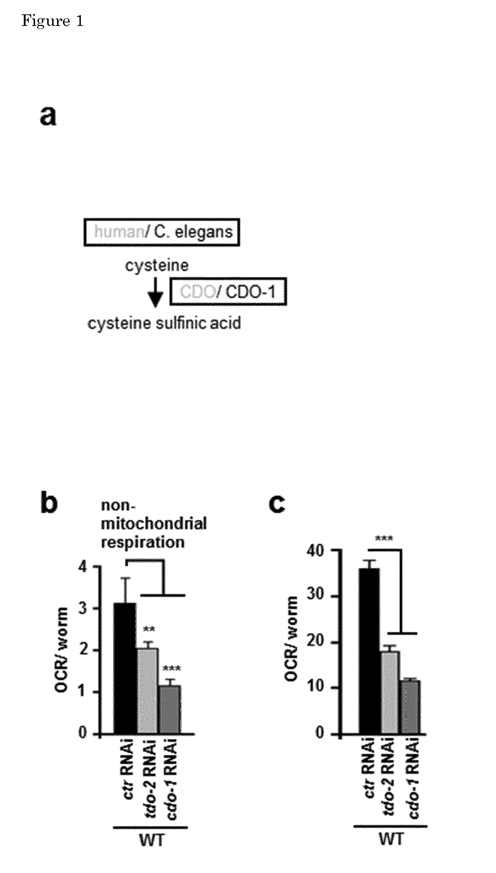

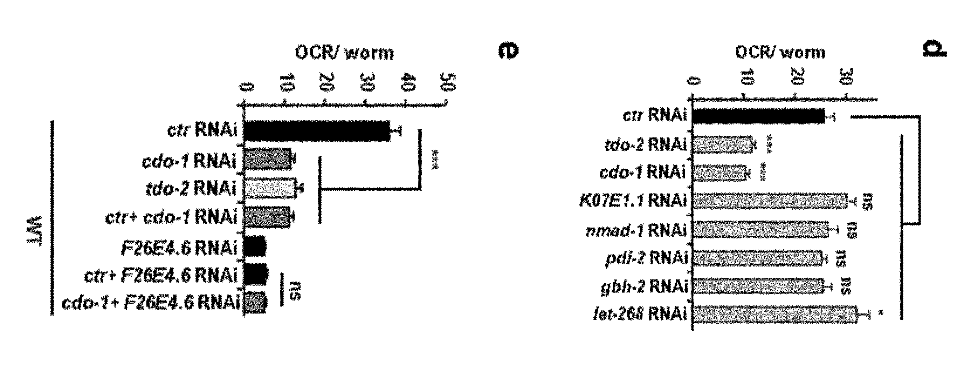

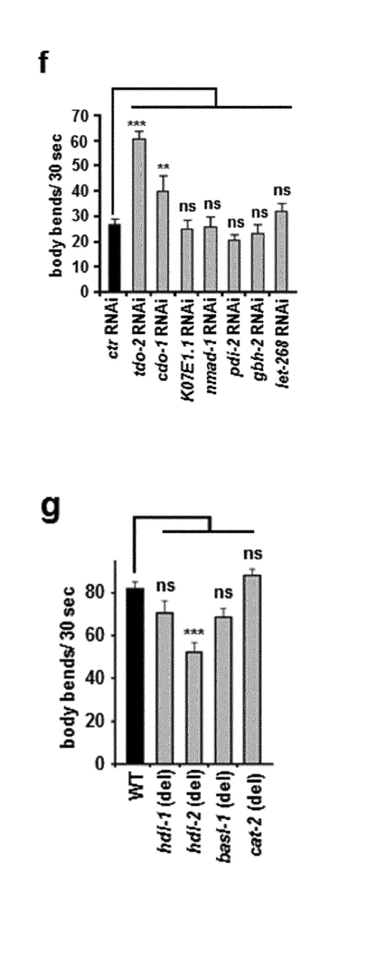

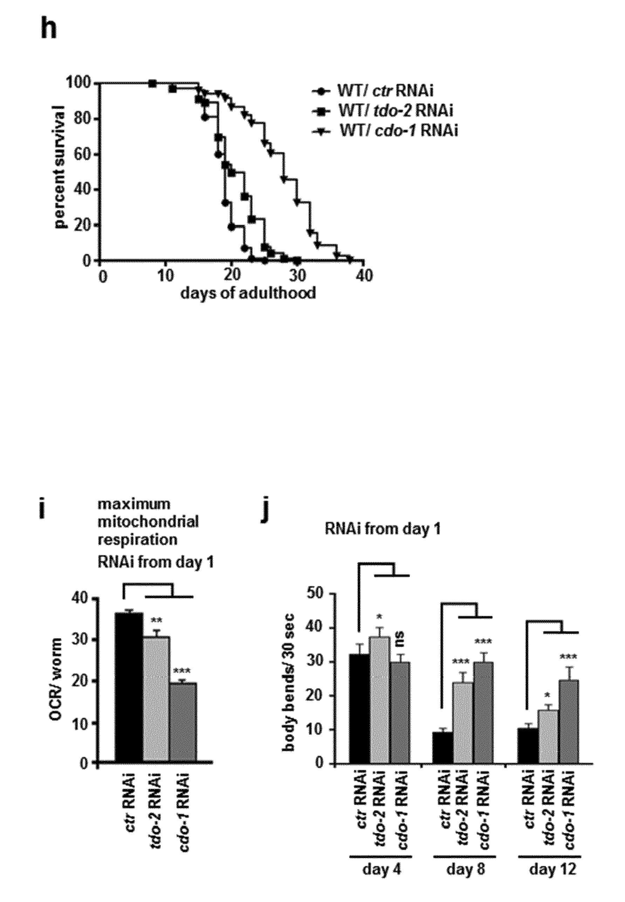

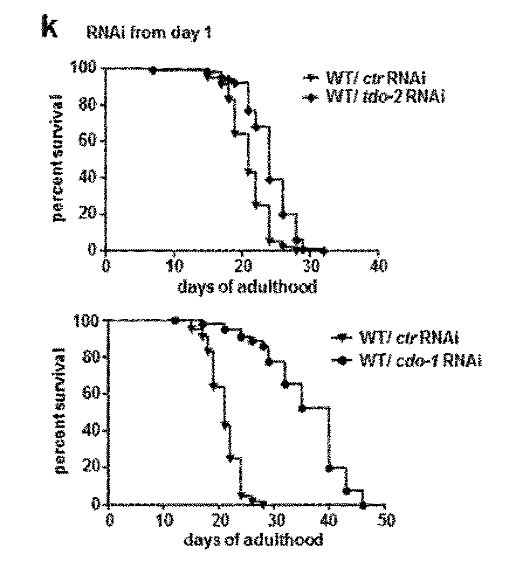

### Figure 1. CDO-1 has cellular functions in respiration control and health- and lifespan regulation

Panel a: Schematic overview of cysteine degradation by CDO/CDO-1 (cysteine dioxygenase) in human and *C*. *elegans.*
Panel b: Non-mitochondrial oxygen consumption (OCR) on day 4 of adulthood.
Panel c: Maximum mitochondrial oxygen consumption rate (OCR) on day 4 of adulthood.
Panel d: Maximum mitochondrial capacity on day 4 of adulthood upon depletion of oxygen-consuming enzymes.
Panel e: Maximum mitochondrial capacity on day 4 of adulthood upon depletion of *tdo-2* and *cdo-1* in combination with complex IV subunit F26E4.6
Panels f and g: Motility/healthspan of oxygen-consuming (f) and amino acid-degrading enzymes (g) on day 4 of adulthood.
Panel h: Survival curves of *cdo-1*-depleted animals.
Panel i: Maximum mitochondrial capacity on day 4 of adulthood, RNAi from day 1 onwards.
Panel j: Motility/healthspan, RNAi from day 1 onwards.
Panel k: Survival curves in response to depletion of *tdo-2* and *cdo-1* from day 1 of adulthood onwards. Statistics for all panels: ns = not significant, * <0.05, ** <0.01, *** <0.001. Error bars = SEM.

### EXPERIMENTAL SECTION

### Media and strains

Nematodes were grown at standard conditions at 20°C. Animals were age-synchronized by hypochlorite treatment, hatched overnight in M9 buffer and subsequently cultured on Nematode Growth Medium (NGM) seeded with *Escherichia coli* strain OP50. On day 1 of adulthood (one day after larval stage L4), animals were transferred to NGM plates containing 5-fluoro-2'deoxy-uridine (FUDR) to prevent the offspring from growing. The wildtype N2 strain was used in this study.

### RNA interference

RNAi experiments were performed on NGM plates containing isopropylthio-ß-D-galactoside (IPTG, 15mg/L) and 50µg/ml ampicillin. Plates were seeded with RNAi bacteria. Prior to the experiment, the plates were kept at room temperature for 2 days to allow the production of dsRNA by the bacteria. Plates were produced as freshly as possible (maximum 1 week before start of the experiment). Age-synchronized animals were either grown directly on RNAi food from larval stage L1 onwards or transferred to the RNAi treatment at larval stage L4 or day 1 of adulthood (indicated for specific experiments). For each experiment, the timepoint at which RNAi treatment started was determined depending on the results of a test run to see whether such treatment affected development. Animals of day 1 of adulthood (day after L4 stage) were transferred to RNAi plates containing FUDR to prevent the offspring from growing. RNAi clones were used from the Ahringer *C. elegans* RNAi library (Fraser et al. Nature 408, 325-30 (2000) unless otherwise indicated (see below). All clones were verified by sequencing and animals were scored for associated phenotypes during the experiments.

The *cdo-1* RNAi clone was generated by a nested PCR. For the first PCR forward primer 5'-gcctaaaaaaccgtgttctgc-3' and reverse primer 5'-ggatgtgatccgacttctacagg-3' were used. The follow-up PCR used forward primer 5'- gttcaaattcgtgaaatcttcc-3' and reverse primer 5'-ctgtagtcaactttcttgccg-3'. The PCR product was cloned into the L4440 vector and transformed into HT115 bacteria. Cloning was confirmed by sequencing.

### Motility assay

Animals were scored at day 4 of adulthood unless otherwise indicated. Animals were placed in a drop of M9 and allowed to adjust for 30 sec, after which the number of body bends was counted for another 30 sec. 15 animals were scored per experimental group. Treatments respectively mutations were blinded before counting body bends. Assays were repeated in three independent experiments and the data of one representative experiment is shown.

### Seahorse respiration studies

Oxygen consumption rates (OCR) were measured using the Seahorse XF96 cycler on day 4 of adulthood unless otherwise indicated. OCR was measured as pMoles/min/worm. Respiration behaviour was optimized for wild type and *tdo-2* deletion mutants at room temperature. The following cycles were used (1 loop = mixing: 2 minutes, waiting: 30 sec, measuring: 2 minutes): (1) calibration (2) equilibration (3) basal respiration = measurement for 5 loops (4) maximum mitochondrial capacity = first injection of FCCP, then measurement for 9 loops (5) non-mitochondrial respiration = first injection of sodium azide, then measurement for 4 loops. Mitochondrial OCR was determined by subtracting non-mitochondrial OCR from basal respiration levels. Maximum mitochondrial capacity was calculated by subtracting non-mitochondrial OCR from maximum FCCP-induced respiration. Paraquat was injected after measurement of basal respiration levels and before injection of FCCP and measured for six loops. The following concentrations were used for injection: 20µl of 100µM FCCP, 20µl of 400mM sodium azide and 20µl of 200mM paraquat. Each experimental group was tested in 6-8 wells, each containing 10-30 worms/well (counted afterwards). Only conditions of the same run can be compared. Experiments were repeated and one representative graph is shown with error bars = SEM.

### Lifespan experiments

Experiments were performed at 20°C. 100 animals per experimental group were tested in one single experiment. Lifespan assays were repeated independently and one representative curve is shown. Animals were treated with RNAi starting from larval stage L1 onwards unless otherwise indicated. Animals were transferred to FUDR-containing plates on day 1 of adulthood. At each time point, the surviving animals were counted and dead animals removed (those no longer responding to nose touch). Animals that disappeared during the assay were excluded from the analysis. Treatments respectively mutations were blinded before starting the experiment. Lifespan curves were generated using GraphPad Prism software.

### Statistics

Statistical analysis for lifespan experiments and stress assays was performed with a log-rank Mantel-Cox test. Data comparison for motility/healthspan as well as for respiration experiments was done by a one-way ANOVA with post-hoc Bonferroni algorithm. If relationships are not otherwise indicated, p values reflect a comparison with the control or wild type conditions.

### RESULTS

Tryptophan 2,3-dioxygenase (TDO-2; TDO in human) is a haem-containing, oxygen-dependent, cytosolic enzyme that degrades tryptophan into formylkynurenine as a first step in the kynurenine pathway (Schwarcz et al., Nat Rev Neurosci 13(7), 465-477 (2012). TDO thus regulates systemic levels of tryptophan, an essential amino acid and a precursor of other biogenic amines, including serotonin and tryptamine (reviewed in Van der Goot et al. Trends Mol Med 19(6), 336-44 (2013)). Higher levels of TDO activity are thought to be involved in various age-related diseases. For example in certain cancers, a high TDO activity is believed to cause tumoral immune tolerance (Pilotte et al., Proc Natl Acad Sci USA 109(7), 2497-502 (2012) and Opitz et al. Nature 478(7368), 197-203 (2011); in type 2 diabetes, patients with high plasma kynurenine levels are more likely to have severe insulin resistance (Oxenkrug Mol Neurobiol 52(2), 805-10 (2015); and patients with neurodegenerative diseases have been found to have an imbalance in kynurenine metabolites. (Guidetti et al., Neurobiol Dis 17(3), 455-461 (2004); Stoy et al. J Neurochem 93, 611-623 (2005); Wu et al.: PLoS One 8(4), (2013).

Because the benefits of TDO-depletion for lifespan and neurodegenerative proteotoxicity are independent of the kynurenine-pathway, we performed transcriptome profiling in TDO-depleted *C*. *elegans* worms to find kynurenine-independent roles of TDO-2. These studies revealed that TDO-2 depletion upregulates genes involved in mitochondrial beta-oxidation. Experimental TDO-2 depletion in the animals reduced their mitochondrial oxygen consumption and ATP levels, suggesting that TDO-2 regulates mitochondrial function. This effect was confirmed to be independent of the kynurenine pathway and other known tryptophan-related pathways (data not shown).

In search for which other aspects of TDO-2's enzymatic activity might be responsible for the mitochondrial phenotype observed, we depleted other amino acid dioxygenases, including CDO-1 (cysteine dioxygenase, CDO in human), which degrades cysteine (Fig. 1a) in *C. elegans.*

Surprisingly, depletion of *cdo-1* also resulted in reduced non-mitochondrial (Fig. 1b) and maximum mitochondrial oxygen consumption (Fig. 1c), similar to that observed for the *tdo-2(del)* mutants. Importantly, the effect of *cdo-1* inhibition was very strong as compared to the other depletions, thus indicating a prime role for this enzyme.

In contrast, depletion of enzymes that only consume oxygen or that only degrade amino acids had no effect on mitochondrial respiration (Fig. Id). Thus, inhibition of the enzyme cysteine dioxygenase (cdo-1), reduced mitochondrial capacity and phenocopied the health- and lifespan extension seen for TDO-2 depletion (Fig. 1f,h). The fact that other enzymes that either are oxygen-dependent (Fig. If) or degrade amino acids (Fig. 1g) did not share this role in health span regulation points to a specific function for amino acid dioxygenases, combining oxygen use with amino-acid conversion. The effect was also seen when cdo-1 was knocked down at day one of adulthood, i.e. after development is complete (see Fig. 1i-k).

## Claims

1. A method of prolonging the lifespan of a subject or preventing or reducing age-related symptoms, comprising:
administering an agent that inhibits the conversion of cysteine to cysteine sulfinic acid to a subject, wherein said administering prolongs the lifespan of said subject relative to the lifespan in the absence of said agent or wherein age-related symptoms are reduced or prevented.

2. Compound for use in a method of prolonging the lifespan of a subject or preventing or reducing age-related symptoms in a subject, said method comprising administering to said subject an agent that inhibits the conversion of cysteine to cysteine sulfinic acid, wherein said administering prolongs the lifespan of said subject relative to the lifespan in the absence of said agent or wherein age-related symptoms are reduced or prevented.

3. Method or compound for use according to claim 1, wherein said agent inhibits cysteine dioxygenase (CDO).

4. Method or compound for use according to any one of claims 1 to 3, wherein said agent is selected from the group consisting of a small molecule, a nucleic acid, and an antibody.

5. Method or compound for use according to any one of claims 1 to 4, wherein said administering treats or prevents one or more aging-associated medical or psychiatric disorders.

6. Method or compound for use according to claim 5, wherein said disorder is a neurodegenerative disorder.

7. Method or compound for use according to claim 6, wherein said disorder is Huntington's disease, Parkinson's disease or Alzheimer's disease.

8. A method of identifying a compound capable of prolonging the lifespan of a subject or preventing or reducing age-related symptoms in a subject, comprising:
a) contacting a cell with a test compound; and
b) identifying a test compound that inhibits the conversion of cysteine to cysteine sulfinic acid

9. The method of claim 8, comprising determining whether said test compound inhibits cysteine dioxygenase (CDO).

10. The method of claim 8 or 9, wherein said cell is a mammalian cell, preferably a human cell.

11. The method of claim 10, wherein said cell is in an animal, preferably wherein said animal is *C. elegans.*

12. The method of claim 8-11, wherein said test compound is selected from the group consisting of a small molecule, a nucleic acid, and an antibody.

13. A pharmaceutical composition comprising an agent that inhibits the conversion of cysteine to cysteine sulfinic acid, preferably an inhibitor of CDO, and a pharmaceutically acceptable carrier, diluent or vehicle.

14. A composition, comprising:
a) a first agent that inhibits the conversion of cysteine to cysteine sulfinic acid; and
b) a second agent known to be useful in prolonging the lifespan of a subject or preventing or reducing age-related symptoms.

15. The composition of claim 14, wherein said first agent inhibits CDO.
